# EUROPEAN PATENT APPLICATION

(11) **EP 4 372 756 A1**
(43) Date of publication of application: **22.05.2024**
(21) Application number: 22290085.4
(22) Date of filing: 15.11.2022
(51) Int. Cl.: G16H 30/20, G16H 30/40

(54) **COMMUNICATING MEDICAL IMAGES**

(71) Applicant: Koninklijke Philips N.V., 5656 AG Eindhoven (NL)
(72) Inventor: De Craene, Mathieu, 5656 AE Eindhoven (NL); Allain, Pascal, 5656 AE Eindhoven (NL); Huiberts, Johannes Nicolaas, 5656 AE Eindhoven (NL); Matters, Marco, 5656 AE Eindhoven (NL)
(74) Representative: Philips Intellectual Property & Standards

(57) **Abstract**

The invention relates to a prioritized communication protocol based on a score of medical image data sets, the protocol comprising: receiving (2020) a plurality of medical image data sets; scoring (2040) at least two of the plurality of medical image data sets; sorting (2060) the plurality of medical image data sets; and communicating (2080) at least a subset of the plurality of medical image data sets based on the sorting of the plurality of medical image data sets, in that a better scoring medical image data set is communicated before a worse scoring medical image data set. This protocol enables, amongst others, an efficient communication between a medical imaging device (2020) acquiring medical images and a receiving storage (2100), where the most relevant medical image data sets may be reviewed without frustrating wait times.

## Description

### FIELD OF THE INVENTION

The invention relates to communicating medical images, and more particularly to prioritizing images in the communication based on an image score.

### BACKGROUND OF THE INVENTION

Medical imaging data is often communicated between the medical image acquisition devices and a local or remote archiving system for storage (e.g., a Picture Archiving and Communication System PACS), processing system for quantification or display system for further visual review. In conventional implementations, this communication takes place in the order of data acquisition, (i.e., in the order medical images are acquired during a medical imaging exam) or in any random order. In some instances, however, a medical practitioner may wish to access the medical imaging data in (near) real-time, or shortly after the exam, when not all images have been communicated. In these instances, it can be frustrating to wait for the full upload of the medical imaging data before being able to further process the data. The hardware of the involved systems may be optimized, and or replaced by newer more optimized components, but this is not always possible, nor feasible. Software solutions are thus highly sought after to improve the communication workflow between medical imaging acquisition devices and local or remote archiving systems, processing systems or displays.

### SUMMARY OF THE INVENTION

It is, inter alia, an object of the invention to provide a medical imaging communication framework to prioritize the communication queue according to an image score. In this way, important images (e.g., images with a higher score) may be communicated before less important images (e.g., images with a lower score). The image score may amongst other things include image quality, presence or absence of specific landmarks (e.g., organs, tissue, implants), relevance for a medical procedure and adequacy to be included or referenced in a medical report.

The invention is defined by the independent claims. Advantageous embodiments are defined in the dependent claims.

In an aspect of the present invention, a computer-implemented method for communicating medical image data sets is provided, the method comprising:
- receiving a plurality of medical image data sets;
- determining a score for at least two medical image data sets in the plurality of medical image data sets;
- sorting the plurality of medical image data sets based on the score; and
- communicating at least a subset of the plurality of medical image data sets based on the sorting of the plurality of medical image data sets, in that a better scoring medical image data set is communicated before a worse scoring medical image data set.

In this manner, medical images are communicated based on a sorting of the medical images, such that a receiving system or device (e.g., a receiving archiving system, a receiving display, a receiving processing unit) may receive first the most relevant images. For example, a first physician may be performing a medical imaging examination, and a second physician at the receiving end may review the most relevant examination images during the examination, or shortly thereafter, with minimal waiting time, thus improving workflow. In an example, the communication may be used in any medical imaging modality when data transfer is limited, thus choosing to transmit relevant images. In an example, a physician may wish to have the medical images analyzed/examined by a processing unit comprising any algorithm to review the medical images. In this example, the prioritized communication of medical images may improve the workflow by communicating first relevant images for said reviewing algorithm.

The communicating step may thus comprise transmitting or sending the medical image data sets from one device or system to another device or system. For example, medical image data sets may be communicated from a medical imaging device to a local or remote server (cloud), or to a remote display. For example, in X-ray imaging the medical image data sets may be communicated from the imaging system to a PACS server. In an example, the medical image data sets may be communicated wired or wirelessly from a hand-held ultrasound device to a desktop or the cloud.

The sorted list of medical image data sets may also be called a communication queue, wherein the highest scoring medical image data set is communicated before a lower scoring medical image data set.

In an example, the score comprises any one of:
- a medical image data set quality assessment,
- a feature detection assessment,
- a conformity with a standard view assessment,
- a relevance for a medical report assessment, and
- a change in condition in the medical image data set with respect to a previous medical image data set, wherein the condition is an injury or any abrupt change in an index derived from the image data set.

In an embodiment the medical image data set quality is assessed by
- a trained machine learning algorithm and/or
- an external user, and
the image data set quality comprises any one of:
- speckle quality,
- contrast assessment,
- signal to noise ratio, and
- image artefacts.

In an embodiment the feature detection is carried out by
- a trained machine learning algorithm or
- by segmentation; and
the detected features comprise any one of:
- procedure specific landmarks,
- pathological features,
- organs, and
- implants.

In some examples, the method above further comprises:
- receiving an indication of a medical imaging procedure, wherein the indication is received:
   - automatically based on the feature detection, or
   - based on an external user input;

   wherein the conformity with a standard view is determined based on a standard view of the received medical imaging procedure and/or
   wherein the relevance for a medical report is determined based on a check list of images to be completed for the received medical imaging procedure.

For example, an external user may select a particular medical treatment from which a medical imaging procedure or protocol follows. In an example, the external user may select a medical imaging procedure in accordance with an insurance protocol. For example, an insurance may have requirements on particular views of an organ for reimbursement.

In some embodiments, the medical image data sets may be embedded with an additional label or tag, that identifies the medical image data sets as relevant for a medical diagnostic or billing report. In this way, images may be selected at a receiving end to generate an automatic report suitable for billing.

According to an embodiment of the present invention, the above method is carried out and repeated during a live imaging examination; and the sorting step is updated during the live imaging examination. In this event the communicating of the medical image data sets may start immediately upon the first image acquisition, and the communication queue (i.e., the images not currently being actively communicated) be updated upon every new entry of a medical image data set. This way, when a new medical image data set is gathered and scored, it is placed in a sorted manner within the communication queue.

In an example, the resolution of the medical image data set to be communicated is adjusted globally based on the score of that medical image data set. In an example, the resolution of the medical image data set to be communicated is adjusted locally based on a detected or user selected region of interest.

In an embodiment, the method further comprises thresholding the score so as to only communicate a subset of medical image data sets of the plurality of medical image data sets for which the score exceeds the threshold. In this manner, medical image data sets that do, for example, not suffice in imaging quality, may be removed from the communication queue so as to minimize the total amount of data to be communicated. Similarly, medical images deemed unsuitable in for example reporting, billing or for analysis by a specific reviewing software may not be communicated.

In some examples, the score may be determined or based on an external user input. For example, if a user disagrees with a specific score or wishes to modify the sorting of the communication queue, the user may overwrite a given score.

According to an embodiment, the medical image data sets comprise medical images. For example, the medical image data set may comprise any one of an ultrasound image, a magnetic resonance (MR) image, a computed tomography (CT) image or an X-ray image, or any other type of medical image.

According to an embodiment, the medical image data sets comprise medical imaging sequences each including a plurality of medical images. For example, in some medical imaging examinations, medical image loops may need to be evaluated, e.g., a heart rhythm or blood flow in an artery during an ultrasound examination, or brain activity during MR imaging.

In an aspect of the present invention, there is provided a processing apparatus configured to perform the above method. The processing apparatus may further comprise
an imaging device configured to obtain the plurality of medical image data sets. The processing apparatus may yet further comprise a display device configured to display medical images. The display may further comprise a graphical user interface for interaction with a user. For example, to set the score and/or correct the score according to the above method.

In an aspect of the present invention there is provided a computer-program comprising instructions, which when executed by a processor, enable the processor to carry out the above method.

These and other aspects of the invention will be apparent from and elucidated with reference to the embodiments described hereinafter.

### BRIEF DESCRIPTION OF THE DRAWINGS

Fig. 1 shows an exemplary flow chart of a method according to an embodiment of the invention.
Fig. 2 shows an annotated training image for a standard view assessment.
Fig. 3 shows an example image where a detected feature is displayed.
Fig. 4 shows an embodiment of a system in accordance with an embodiment of the invention.
Fig. 5 shows how a score is determined for a medical imaging sequence in accordance with an embodiment of the invention.
Fig. 6 shows a use case of a method in accordance with an embodiment of the invention.
Fig. 7 shows a schematic diagram of an ultrasound imaging system according to an embodiment of the present invention.
Fig. 8 shows a schematic of a processor circuit for use in embodiments of the invention.

### DETAILED DESCRIPTION OF EMBODIMENTS

The invention will be described herein with reference to the Figures. It should be understood that the description and specific examples provided, while indicating exemplary embodiments, are intended for purposes of illustration only, and not intended to limit the scope of the invention. It should also be understood that the figures are mere schematics and not drawn to scale. It should also be understood that the same reference numerals are used throughout the Figures to indicate the same or similar parts.

The invention provides a computer-implemented method and system to carry out said method, of communicating medical image data sets in a prioritized manner.

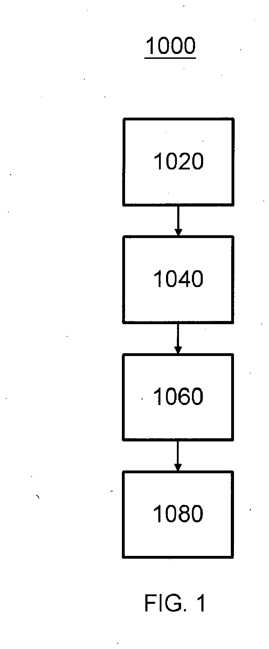

Fig. 1 shows an exemplary flow chart of a method 1000 according to an embodiment of the invention.

In a first step 1020 of the method, a plurality of medical image data sets is received. The medical image sets may be received from a storage device containing medical image data sets or during a medical image examination procedure from a medical image acquisition device such as, without limitation, an ultrasound probe, X-ray detector/source, Magnetic Resonance (MR) scanner and a Computed-Tomography (CT) scanner. The medical image data sets may accordingly also comprise any type of medical image data including medical images and medical imaging sequences, wherein the medical imaging sequences each include a plurality of medical images. The medical image data sets may be annotated a priori before reception in step 1020. The medical image data sets may further be preprocessed, including image and/or image sequence enhancements. Alternatively, the medical image data sets may be raw data.

In an example, the medical image data sets may be medical images (e.g., X-ray images, ultrasound images, CT images, etc.)

In an example, the medical image data sets may be medical imaging sequences (e.g., MR medical imaging sequences, ultrasound medical imaging sequences, etc.)

In a second step 1040 of the method, a score is determined for at least two medical image data sets in the plurality of medical image data sets. Upon reception of the plurality of medical image data sets in step 1020, the method scores some or all of the plurality of medical image data sets according to a pre-defined set of criteria. A known method to assign a score to medical image and then save that image are disclosed in, for example, US 2020/0245976.

In an example, the medical image data set, which may comprise medical images and/or medical imaging sequences, wherein each of the medical imaging sequences comprises a plurality of medical images, may be scored in several ways.

A first way of determining a score for the medical image data sets is based on general medical image data set quality criteria such a sharpness, speckle quality, contrast assessment, signal to noise ratio and local and global image artefacts (e.g., reverberation, aberration, motion artefacts). The medical image data set quality may be assessed by a trained machine learning algorithm and/or an external user. In an example, the user may overwrite an automatically generated medical image data set quality assessment.

In an example, the medical image data set score may thus be based an image quality assessment.

A second way of determining a score for the medical image data sets is based on feature detection assessment. For example, a feature detection assessment may automatically detect the presence and/or absence of organs (e.g., heart, lungs, kidney, etc.), implants and any other specific landmarks or features in the medical image data sets (e.g., pericardial effusion, which is the buildup of fluid in the pericardial cavity, a cyst, etc.).

The feature detection assessment may be based on a medical imaging procedure (e.g., kidney exam, lung exam, etc.), wherein the medical imaging procedure may be received *a priori,* i.e., before the start of the scoring of the medical image data sets. The medical imaging procedure may thus be received based on an external user input. In an example, a medical imaging procedure may be detected and selected automatically based on a feature detection assessment. For example, the feature detection assessment may detect a heart and select a particular cardiovascular medical imaging procedure.

In an example, a list of possible medical imaging procedures may be given to an external user to be selected based on the feature detection assessment. The feature detection assessment may be carried out by a conventional machine learning algorithm. For this, a machine learning algorithm may involve the identification and localization of known landmarks (i.e., organs, implants, etc.) and/or regions of interest (ROIs) (in certain frames for a video).

In an example, the feature detection assessment may be carried out by a user. For example, the user may annotate the medical image data set.

In an example, the medical image data set score may thus be based on a feature detection assessment.

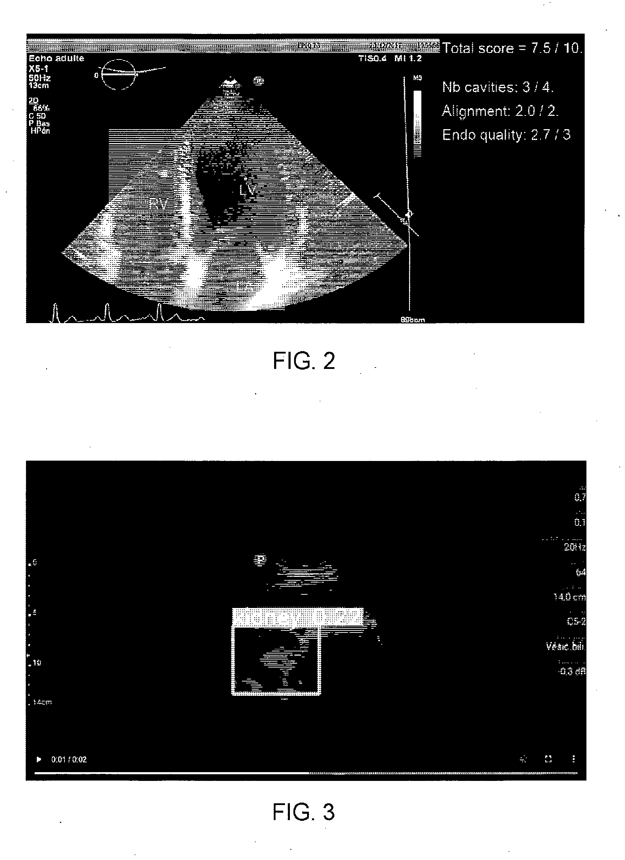

A third way of determining a score for the medical image data sets is based on conformity with a standard view assessment. For example, a machine learning algorithm may involve the localization of known landmarks (i.e., organs, implants, etc.) and/or regions of interest (ROIs) (in certain frames for a video). The relative position of the different landmarks and/or ROIs can then be used to verify that it matches the definition of a standard view. An example is shown in Fig. 2 where the four cardiac chambers are identified (the left and right atria (LA, RA) and the left and right ventricles (LV, RV)). Both their (relative) position and their alignment allow to verify if it corresponds to the layout of a four chambers view (left ventricle and atrium aligned in the right part of the image, right ventricle and atrium aligned in the left part of the images). Such a machine learning algorithm may be trained in a conventional way, including training images annotated like the one in Fig. 2.

The conformity with a standard view assessment may further be based on a medical imaging procedure (e.g., kidney exam, lung exam, etc.)., wherein the medical imaging procedure may be received *a priori,* i.e., before the start of the scoring of the medical image data sets. The medical imaging procedure may thus be received based on an external user input. In an example, a medical imaging procedure may be detected and selected automatically based on a feature detection assessment. For example, the feature detection assessment may detect a heart and select a cardiovascular medical imaging procedure.

In an example, a list of possible medical imaging procedures may be given to an external user to be selected based on the feature detection assessment. Examples of medical imaging procedures may be for example triage protocols.

In an example, the conformity with a standard view may be assessed by a user. For example, the user may assess whether an image data set conforms with a standard view and the user may in response to his/her assessment assign a respective score.

In an example, the medical image data set score may thus include a conformity with a standard view assessment.

A fourth way of determining a score for the medical image data sets is based on relevance for a medical report assessment. For example, physicians (e.g., radiologist, sonographer, doctor or any other form of medical practitioner), medical institutions (e.g., hospitals, clinics, general practitioners' offices, etc.) and/or insurances may have preferences and/or requirements of images to add in a report. The relevance for a medical report assessment may identify these preferences and/or requirements, and automatically consider these when determining the score. In an example, the relevance for a medical report is determined based on a check list of images to be completed for a received medical imaging procedure and/or a received medical reporting template. For example, an insurance may require three particular image views in lung ultrasound for billing. A machine learning algorithm may thus consider the relevance of images in order to communicate the three required image views image views. A relevance for a medical report assessment may also include user preferences based on selected settings or based on a user history, such that it may be determined how likely a given medical image data set is to be included in a medical report.

The medical report assessment may further be based on a medical imaging procedure and/or medical report template, wherein the medical imaging procedure and/or the medical report template may be received *a priori,* i.e., before the start of the scoring of the medical image data sets. The medical imaging procedure and/or medical report template may thus be received based on an external user input. In an example, a medical imaging procedure and/or medical report template may be detected and selected automatically based on a feature detection assessment. For example, the feature detection assessment may detect a heart and select a cardiovascular medical imaging procedure.

In an example, a list of possible medical imaging procedures and/or medical report templates may be given to an external user to be selected based on the feature detection assessment.

In an example, a user may determine the relevance for a medical report for the medical image data set. The user may further assign a score for the relevance for a medical report.

In an example, the medical image data set score may thus include a likelihood for a medical report assessment.

It is to be understood that a medical imaging procedure and/or report template may contain a protocol to be followed by a physician during imaging. In an example, this protocol may be a protocol of for example a standard kidney exam (e.g., include left and right kidney in both short and long axes). In an example this protocol may include a list of required images for billing at a particular insurance. Such protocols may also be created by a user. For example, a particular user always wants a frontal X-ray image of a hip implant.

A fifth way of determining a score for the medical image data sets is based is based on determining a change in condition. For example, the progress of an injury and/or disease may be monitored in for example ultrasound images. For example, in lung X-rays of a subject during the course of the subject having pneumonia. A score may be assigned according to the observed changes by a machine learning algorithm such as the one presented by Li et al. (2020) "Siamese neural networks for continuous disease severity evaluation and change detection in medical imaging" or a score may be assigned by a user.

In a third step1060 of the method, the plurality of medical image data sets is sorted based on the score. For example, the plurality of medical image data sets is sorted from highest scoring to lowest scoring medical image data set. This list may also be called a communication queue. In an example when not all medical image data sets are scored, the not-scored medical image data sets may be excluded from the communication queue, or assigned a score of zero. In an example when not all medical image data sets are scored, the not-scored medical image data set are assigned a score based on the score of medical image data sets acquired before and/or after the not-scored medical image data set. For example, if three medical images are acquired at time. steps 1, 2 and 3, and only medical image 1 and 3 are scored, medical image 2 may be assigned an average score based on the scored of medical images 1 and 3.

In a fourth step 1080 of the method, at least a subset of the plurality of medical image data sets is communicated based on the sorting of the plurality of medical image data sets, in that a better scoring medical image data set is communicated before a worse scoring medical image data set. For example, the communication queue obtained in step 1060 is communicated to a receiving system or device. The communication may be wired and/or wireless and may be carried out via any known communication technology. For example, the communication may use a wired link, such as a universal serial bus (USB) link or an Ethernet link. Alternatively, the communication may use a wireless link, such as an ultra-wideband (UWB) link, an Institute of Electrical and Electronics Engineers (IEEE) 802.11 WiFi link, or a Bluetooth link. In other words, the communication may comprise a transmission, sending and/or uploading of medical image data sets to another device or system.

The receiving system or device may be any system suitable for storage, visualization, further processing and or external review of the medical image data sets. In an example, the receiving system may be a Picture Archiving and Communication system. In an example the receiving system may be a display. In an example the receiving system may be a processing system.

In an example, the method 1000 may further include the ability to send push notifications and request review of the communicated medical image data sets.

In an example, the method 1000 may further include the ability to request referral of a subject or to refer a subject.

In an example, the method 1000 may further include receiving feedback from an external user, wherein the feedback is in reply to the external user receiving the communicated medical image data set.

According to the method 1000, medical image data sets are thus received and scored based on their importance with respect to a pre-defined set of criteria, (e.g., image data set quality, presence or absence of particular features, conformity with standards and relevance for a medical diagnostic or billing report), and are communicated to a local or remote receiving system or device based on the score, in that better scoring medical image data sets are transmitted before worse scoring medical image data sets.

In an example, the medical image data set score may further be thresholded so as to only communicate a subset of medical image data sets of the plurality of medical image data sets for which the score exceeds the threshold. In this manner medical image data sets unsuitable for further review due to for example poor image quality, may be removed entirely from the communication queue.

In an example, the individual components of the score (e.g., medical image data set quality, feature detection assessment, conformity with a standard view and relevance for a medical report) may be combined and may comprise different weights. In an example, these weights may be set by a user. For example, a user may for example rank relevance for reporting as the top priority and use this as the sole criteria, or assign it a higher weight when determining the medical image data set score. For example, a user may find the relevance for a medical report component of little relevance and assign it a weight equivalent to zero, so that it is not considered when determining the score. In an example, the weights individual score component weights may be set by a machine learning algorithm. For example, the weights may be learned implicitly by end-to-end training a neural network. For example, the weights may be determined explicitly using optimization. The weights may further be adjusted by a user. In an example the weights of the different score criteria may be adjusted per medical image data set so as to for example transfer medical image data sets required for a particular medical report, where the medical image data sets may otherwise be discarded due to for example poor image quality.

In an example, the score may be based on a single machine learning algorithm and/or user input. In an example each component of the score is determined based on a separate machine learning algorithm for each component and/or a separate user input for each component. In an example, a combination of machine learning algorithms may be used for each component of the score. In an example, a machine learning algorithm may cover various components of the score (e.g., two or three components, for example feature detection assessment, conformity with a standard view and relevance for a medical report). In an example the score may be based on a combination of machine learning algorithms and user inputs.

In an example, the final score and/or sorted list of medical image data sets may be adjusted based on an external user input. For example, a user may set or overwrite the score and/or resort the plurality of medical image data sets. In this manner, a user can force an image to be communicated with a particular priority (e.g., highest, medium or lowest). In particular, a user may be able to select an image data set to be communicated next. The currently being communicated medical imaging data set may be interrupted. For example, by a user, so as to communicate a user selected medical image data set.

In an embodiment, the resolution of the to be communicated medical image data sets may be adjusted based on the medical image data set score. For example, higher scoring medical image sets may be communicated at a higher resolution than lower scoring medical image date sets. In an example, the resolution may be adjusted within a medical image data set. For example, the region of a medical image within a region of interest may be communicated in full resolution, while the region outside of it may be communicated at a reduced resolution. This may be done in a stepwise approach, i.e., two different resolutions, or in a gradient like manner, where the resolution slowly reduces further away from the region of interest. An example of how this embodiment may be implemented in practice is shown in Fig. 3. A detected region, in this case the region of a kidney, may be communicated at a higher resolution, while the region outside the detected region (e.g., kidney) may be communicated at a lower resolution. Known approaches that modify the resolution of images are provided in for example pending US application 63/415,048 (attorneys' reference 2022PF00539), where it is proposed to sacrifice some image quality (i.e., resolution reduction or frame rate reduction) in order to ensure prompt delivery of the information, and thus enabling quicker communication of the medical imaging data. However, the obtained image at the receiving end is not always suitable for quantification or review due to either it being an unsuitable medical image (i.e., blurry, wrong features, etc.), or the resolution having been sacrificed too much.

A skilled person in the art will observe that the score as referred to in method 1000 may be a numerical score (e.g., ranking from 1 to 100, wherein a medical image data set scoring 100 is communicated with the highest priority and a medical image data set scoring 1 is communicated with the lowest priority), or the score may be alphabetic (e.g., A to F (A, B, C, D, E, F), with A representing a good score and F representing a very low score), or the score may be given in terms of color, green, yellow, red. In these cases, a high score is presumed that a high score indicates a high relevance. A person skilled in the art will appreciate that in some examples a low score will indicate a high relevance.

It is understood by a person skilled in the art that while method 1000 was presented in a specific order, the order of the steps may vary, additional steps may be added in between, and/or steps may be removed. It is also understood that the method 1000 may also be carried out during a live medical imaging examination, such that the steps may be repeated in real-time. For example, as new medical image data sets are acquired, some or all of the new medical image data sets may be scored according to step 1040 and the communication queue (i.e., sorted list of the plurality of medical imaging data sets) may be updated in step 1060 according to any set of criteria. In an example the communication queue is updated every time a new medical image data set is acquired or scored. In an example, the communication queue is updated after a pre-determined time lapse. In an example the communication queue is updated after a pre-determined number of image data sets were acquired and/or scored. In an example, the medical image data set is updated based on a user request. In an example the communication queue is updated-based on a local memory limitation.

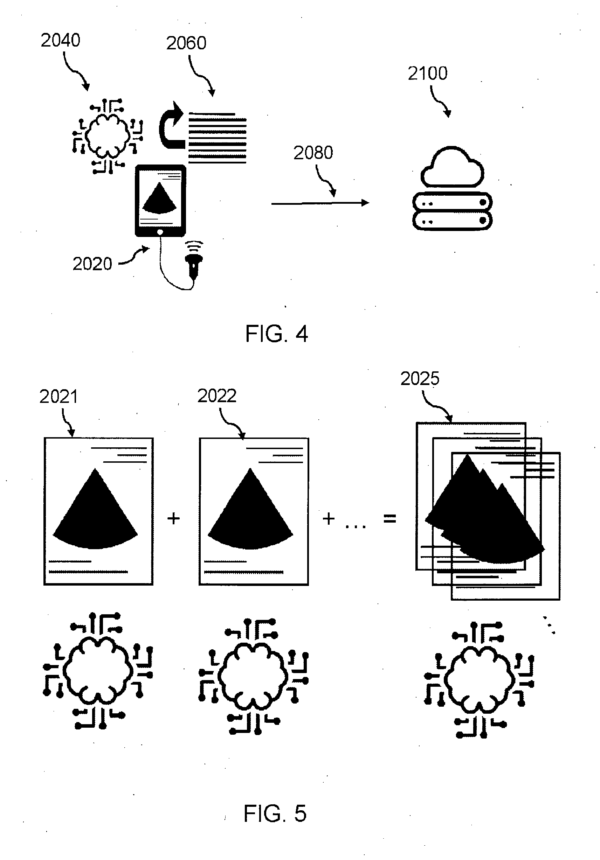

An exemplary system representation of the method 1000 during an ultrasound imaging procedure is provided by Fig. 4 which shows:
- a medical imaging acquisition device 2020 (here a tablet equipped with an ultrasound probe), wherein in the medical imaging acquisition device;
   - a score is assigned to the medical image data sets according to an Image Quality (IQ) Scoring Artificial Intelligence (AI) 2040; and
   - a communication queue is generated, wherein the communication queue comprises a list of sorted medical image data sets 2060 according to the score; and
   a receiving device 2100, to which the medical image data sets are communicated 2080 based on the communication queue, so that a better scoring medical image data set is transferred before a worse scoring medical image data set.

In an example, a user of the medical imaging acquisition device 2020 may also manipulate the medical image data set score and resulting communication queue on a display according to the method 1000. For example, a user may alter the order in the communication queue. For example, a user may assign an own scoring criterion. For example, a user may overwrite a score given to a medical image data set. The user may be enabled to carry out these operations via a graphical user interface. For example, via touch, a mouse or a keypad. In an example, the user may carry out these operations via voice control.

Fig. 5 further displays the case where the medical image data sets comprise a plurality of medical imaging sequences each including a plurality of medical images. In this case, at least two of the medical images 2021, 2022 in each medical imaging sequence 2025 may be scored, and the medical imaging sequence score may be based on the score of the scored medical images in that sequence.

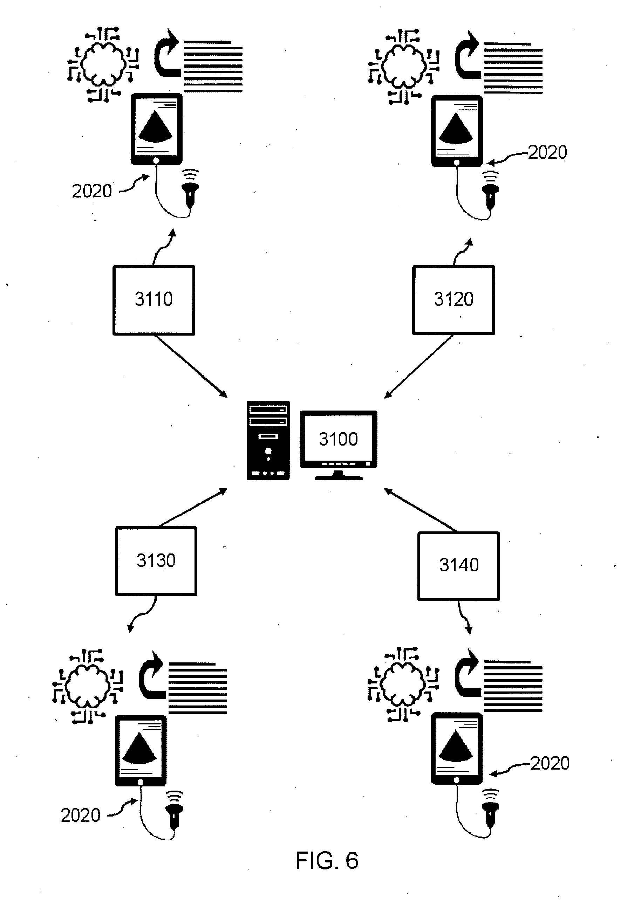

According to an embodiment, the method 1000 may be carried out when training and/or supervising new users (e.g., radiologists, sonographers, physicians, etc.). For example, as shown in Fig. 6, an expert user 3100 may supervise or train a group of users 3110, 3120, 3130, 3140 performing imaging examinations. The expert user may review medical image data sets on a monitoring device that is receiving image data sets from medical imaging devices 2020 used by each the users in the group of users, wherein each of the users in the group of users may be examining a subject with said medical imaging device. By applying the method 1000 to this embodiment, the expert user may thus receive the acquired medical image data sets by each of the users in the group of users based on the communication queue, in such a way that higher scoring medical image data sets are received before lower scoring medical image data sets. Thereby reducing the overall load of medical image data sets to be reviewed by the user, minimizing the data needed to be transferred between the group of users and the expert user, and improving a training and/or supervising workflow. The expert user may further communicate back to the group of users via for example a phone or chat. In the communication the expert user may communicate tips to improve and/or instructions to take a better and/or desired image. This manner of supervising and training physicians for imaging examinations may be used in any kind of medical imaging procedure such as for example ultrasound, X-ray, MR imaging and CT imaging.

According to an embodiment, the method 1000 may be carried out during an ultrasound examination in a point of care setting, where ultrasound images and/or imaging sequences are communicated from a terminal operating the ultrasound probe to a Picture Archiving Communication System (PACS). The method 1000 may be of particular relevance in an Intensive Care Unit (ICU), where quick review of the medical image data sets by various physicians, potentially placed remote from the subject, is necessary. Implementing method 1000, the most relevant images for review may thus be communicated first, reducing the waiting time of each of the physicians waiting to review the images, reducing the time to diagnosis and potentially saving lives. In an example, the method may further include a push notification, wherein a physician performing an imaging exam may request review by a particular other physician. Thereby further increasing the speed toward diagnosis. In an example, a push notification may also be sent out automatically based on a particular finding in a medical image data set.

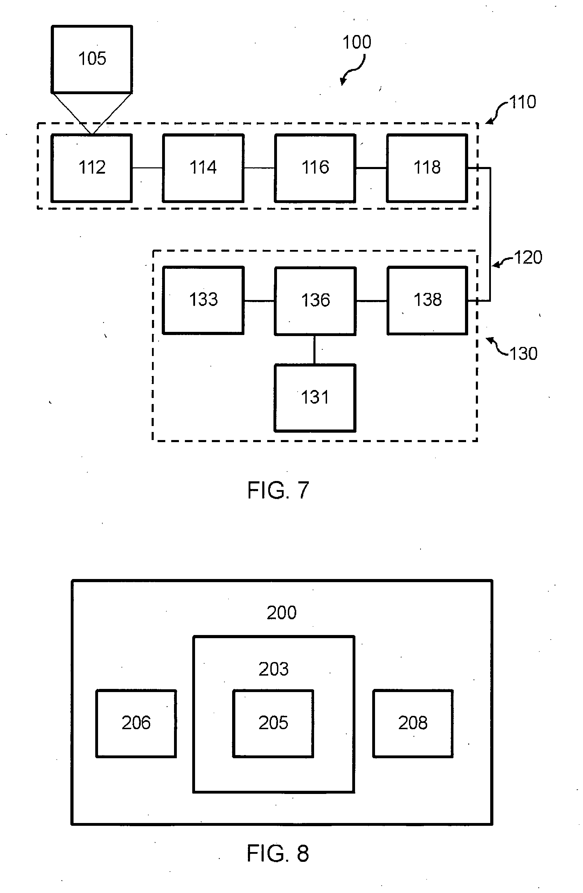

Fig. 7 shows a schematic diagram of an ultrasound imaging system 100 according to an embodiment of the present invention. The system 100 includes an ultrasound imaging probe 110 in communication with a host 130 over a communication interface or link 120. The probe 110 may include a , transducer array 112, a beamformer 114, a processor 116, and a communication interface 118. The host 130 may include a display 131, a processor 136, a communication interface 138, and a memory 133. The host 130 and/or the processor 136 of the host 130 may also be in communication with other, external types of systems or devices in replacement or addition to the here mentioned systems such as for example, an external memory (e.g., a Picture Archiving and Communication System), external display, a subject tracking system, an inertial measurement unit, etc. It is understood that the beamformer may also be a micro-beamformer. It is further understood that the components as shown here may also be configured in alternate arrangements. For example, the processor 116 and/or the beamformer 114 may be located outside of the probe 110 and/or the display 131 and/or memory 133 may be located outside of the host 130.

In some embodiments, the probe 110 is an ultrasound imaging device including a housing configured for handheld operation by a user. The transducer array 112 can be configured to obtain ultrasound data while the user grasps the housing of the probe 110 such that the transducer array 112 is positioned adjacent to or in contact with a patient's skin. The probe 110 is configured to obtain ultrasound data of anatomy within the patient's body while the probe 110 is positioned outside of the patient's body. In some embodiments, the probe 110 can be a patch-based external ultrasound probe. For example, the probe may be a hemodynamic patch.

The transducer array 112 emits ultrasound signals towards an anatomical object 105 of a patient and receives echo signals reflected from the object 105 back to the transducer array 112. The transducer array 112 can include any suitable number of acoustic elements, including one or more acoustic elements and/or a plurality of acoustic elements. In some instances, the transducer array 112 includes a single acoustic element. In some instances, the transducer array 112 may include an array of acoustic elements with any number of acoustic elements in any suitable configuration. For example, the transducer array 112 can include between 1 acoustic element and 10000 acoustic elements, including values such as 2 acoustic elements, 4 acoustic elements, 36 acoustic elements, 64 acoustic elements, 128 acoustic elements, 500 acoustic elements, 812 acoustic elements, 1000 acoustic elements, 3000 acoustic elements, 8000 acoustic elements, and/or other values both larger and smaller. In some instances, the transducer array 112 may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x-dimensional array (e.g., a 1.5D array), or a two-dimensional (2D) array. The array of acoustic elements (e.g., one or more rows, one or more columns, and/or one or more orientations) can be uniformly or independently controlled and activated. The transducer array 112 can be configured to obtain one-dimensional, two-dimensional, and/or three-dimensional images of a patient's anatomy. In some embodiments, the transducer array 112 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

The beamformer 114 is coupled to the transducer array 112. The beamformer 114 controls the transducer array 112, for example, for transmission of the ultrasound signals and reception of the ultrasound echo signals. In some embodiments, the beamformer 114 may apply a time-delay to signals sent to individual acoustic transducers within an array in the transducer array 112 such that an acoustic signal is steered in any suitable direction propagating away from the probe 110. The beamformer 114 may further provide image signals to the processor 116 based on the response of the received ultrasound echo signals. The beamformer 114 may include multiple stages of beamforming. The beamforming can reduce the number of signal lines for coupling to the processor 116. In some embodiments, the transducer array 112 in combination with the beamformer 114 may be referred to as an ultrasound imaging component. The beamformer 114 may also be a micro-beamformer.

The processor 116 is coupled to the beamformer 114. The processor 116 may also be described as a processor circuit, which can include other components in communication with the processor 116, such as a memory, beamformer 114, communication interface 118, and/or other suitable components. The processor 116 is configured to process the beamformed image signals. For example, the processor 116 may perform filtering and/or quadrature demodulation to condition the image signals. The processor 116 and/or 134 can be configured to control the array 112 to obtain ultrasound data associated with the object 105.

The communication interface 118 is coupled to the processor 116. The communication interface 118 may include one or more transmitters, one or more receivers, one or more transceivers, and/or circuitry for transmitting and/or receiving communication signals. The communication interface 118 can include hardware components and/or software components implementing a particular communication protocol suitable for transporting signals over the communication link 120 to the host 130. The communication interface 118 can be referred to as a communication device or a communication interface module.

The communication link 120 may be any suitable communication link. For example, the communication link 120 may be a wired link, such as a universal serial bus (USB) link or an Ethernet link. Alternatively, the communication link 120 may be a wireless link, such as an ultra-wideband (UWB) link, an Institute of Electrical and Electronics Engineers (IEEE) 802.11 WiFi link, or a Bluetooth link.

At the host 130, the communication interface 138 may receive the image signals. The communication interface 138 may be substantially similar to the communication interface 118. The host 130 may be any suitable computing and display device, such as a workstation, a personal computer (PC), a laptop, a tablet, or a mobile phone..

The processor 136 is coupled to the communication interface 138. The processor 136 may ' also be described as a processor circuit, which can include other components in communication with the processor 136, such as the memory 133, the communication interface 138, and/or other suitable components. The processor 136 can be configured to generate image data from the image signals received from the probe 110. The processor 136 can apply advanced signal processing and/or image processing techniques to the image signals. An example of image processing includes conducting a pixel level analysis to evaluate whether there is a change in the color of a pixel, which may correspond to an edge of an object (e.g., the edge of an anatomical feature). In some embodiments, the processor 136 can form a three-dimensional (3D) volume image from the image data. In some embodiments, the processor 136 can perform real-time processing on the image data to provide a streaming video of ultrasound images of the object 105.

The memory 133 is coupled to the processor 136. The memory 133 can be configured to store patient information, measurements, data, or files relating to a patient's medical history, history of . procedures performed, anatomical or biological features, characteristics, or medical conditions associated with a patient, computer readable instructions, such as code, software, or other application, as well as any other suitable information or data. The memory 133 may be located within the host 130. There may also be an additional external memory, or an external memory in replacement of memory 133. An external memory may be a cloud-based server or an external storage device, located outside of the host 130 and in communication with the host 130 and/or processor 136 of the host via a suitable communication link as disclosed with reference to communication link 120. Patient information may include measurements, data, files, other forms of medical history, such as but not limited to ultrasound images, ultrasound videos, and/or any imaging information relating to the patient's anatomy. The patient information may include parameters related to an imaging procedure such a probe position and/or orientation.

The display 131 is coupled to the processor 136. The display 131 may be a monitor or any suitable display or display device. The display 131 is configured to display the ultrasound images, image videos, and/or any imaging information of the object 105.

The system 100 may be used to assist a sonographer or operator in performing an ultrasound scan. The scan may be performed in a point-of-care setting. In some instances, the host 130 is a console or movable cart. In some instances, the host 130 may be a mobile device, such as a tablet, a mobile phone, or portable computer. In yet other examples the host is a server on a cloud and an external display connects to the host in the cloud. During an imaging procedure, the ultrasound system 100 can acquire an ultrasound image of a region of interest of a subject.

Envisaged in the present invention, is the implementation of the method 1000 as part of the communication link 120. For example, in order to minimize data outflow from the ultrasound probe 110. Also envisaged in the present invention, is the implementation of the method 1000 as part of the communication link (not shown) with an external device. For example, to communicate pictures to displays of other users or to a local or remote storage that may be in the cloud, such as a Picture Archiving and Communication System (PACS).

Although only an ultrasound system has been described in detail, a skilled person in the art will find that it is easily possible to also implement the method 1000 in any other medical imaging system or device with the teachings presented here such as for example, but not limited to, X-ray imaging systems, MR imaging systems and CT imaging systems.

The method 1000 may be implemented in any kind of processor circuit. Fig. 8 shows a schematic of a processor circuit 200, according to an embodiment. As shown, the processor circuit 200 may include a processor 206, a memory 203, and a communication module 208. These elements may be in direct or indirect communication with each other, for example via one or more buses.

The processor 206 as envisaged by the present disclosure may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. The processor 206 may also be implemented as a combination of computing devices, e.g., a combination of a DSP and a microprocessor, a plurality of microprocessors, one or more microprocessors in conjunction with a DSP core, or any other such configuration. The processor 606 may also implement various deep learning networks, which may include a hardware or a software implementation. The processor 206 may additionally include a preprocessor in either hardware or software implementation.

The memory 203 as envisaged by the present disclosure may be any suitable storage device, such as a cache memory (e.g., a cache memory of the processor 206), random access memory (RAM), magneto-resistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. The memory may be distributed among multiple memory devices and/or located remotely with respect to the processor circuit. In an embodiment, the memory 203 may store instructions 205. The instructions 205 may include instructions that, when executed by a processor 206, cause the processor 206 to perform the operations described herein.

Instructions 205 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. Instructions 205 may be in the form of an executable computer program or script. For example, routines, subroutines and/or functions may be defined in a programming language including but not limited to C, C++, C#, Pascal, BASIC, API calls, HTML, XHTML, XML, ASP scripts, JavaScript, FORTRAN, COBOL, Perl, Java, ADA, .NET, and the like. Instructions may also include instructions for machine learning, deep learning and/or training of a machine learning module.

The communication module 208 can include any electronic circuitry and/or logic circuitry to facilitate direct or indirect communication of data between the processor circuit 200, and for example an external display (not shown) and/or an imaging device or system such as an X-ray imaging system or an ultrasound imaging system, etc. In that regard, the communication module 208 can be an input/output (I/O) device. The communication may be performed via any suitable communication technology. For example, the communication may use a wired link, such as a universal serial bus (USB) link or an Ethernet link. Alternatively, the communication may use a wireless link, such as an ultra-wideband (UWB) link, an Institute of Electrical and Electronics Engineers (IEEE) 802.11 WiFi link, or a Bluetooth link.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. A computer-implemented method 1000 for communicating medical image data sets, the method comprising:
- receiving (1020) a plurality of medical image data sets;
- determining (1040) a score for at least two medical image data sets in the plurality of medical image data sets;
- sorting (1060) the plurality of medical image data sets based on the score; and
- communicating (1080) at least a subset of the plurality of medical image' data sets based on the sorting of the plurality of medical image data sets, in that a better scoring medical image data set is communicated before a worse scoring medical image data set.

2. The method of claim 1, wherein the score comprises any one of:
- a medical image data set quality assessment,
- a feature detection assessment,
- a conformity with a standard view assessment,
- a relevance for a medical report assessment, and
- a change in condition in the medical image data set with respect to a previous medical image data set, wherein the condition is an injury or any abrupt change in an index derived from the image data set.

3. The method of claim 2,
wherein the medical image data set quality is assessed by
- a trained machine learning algorithm and/or
- an external user, and
wherein the medical image data set quality comprises any one of:
- speckle quality,
- contrast assessment,
- signal to noise ratio, and
- image artefacts.

4. The method of claim 2,
wherein the feature detection is carried out by
- a trained machine learning algorithm, or
- by segmentation; and
wherein the detected features comprise any one of:
- procedure specific landmarks,
- pathological features,
- organs, and
- implants.

5. The method of claim 2 further comprising:
- receiving an indication of a medical imaging procedure, wherein the indication is received:
- automatically based on the feature detection, or
- based on an external user input;
- wherein the conformity with a standard view is determined based on a standard view of the received medical imaging procedure and/or
- wherein the relevance for a medical report is determined based on a check list of images to be completed for the received medical imaging procedure.

6. The method of any of the preceding claims, wherein
- the method is carried out and repeated_during a live imaging examination; and
- the sorting step is updated during the live imaging examination.

7. The method of any of the preceding claims,
wherein the resolution of the medical image data set to be communicated is adjusted globally based on the score of that medical image data set and/or
wherein the resolution of the medical image data set to be communicated is adjusted locally based on a detected or user selected region of interest.

8. The method of any of the preceding claims, further comprising:
- thresholding the score so as to only communicate a subset of medical image data sets of the plurality of medical image data sets for which the score exceeds the threshold.

9. The method of any of the preceding claims, wherein the score is determined or overwritten based on an external user input.

10. The method of any of the preceding claims, wherein the medical image data sets comprise medical images.

11. The method of any of the preceding claims, wherein the medical image data sets comprise medical imaging sequences each including a plurality of medical images.

12. A processing apparatus (116, 136, 200) configured to perform the method of any of claims 1 to 11.

13. The processing apparatus as claimed in claim 12, further comprising:
- an imaging device (110, 2020) configured to obtain the plurality of medical image data sets.

14. The processing apparatus as claimed in claim 12 or 13, further comprising:
- a display device (131) configured to display medical image data sets.

15. A computer-program comprising instructions, which when executed by a processor, enable the processor to carry out the method according to any one of claims 1 to 11.
